# EUROPEAN PATENT SPECIFICATION

(11) **EP 1 732 535 B1**
(45) Date of publication and mention of the grant of the patent: **03.06.2009**
(21) Application number: 04773933.9
(22) Date of filing: 15.06.2004
(51) Int. Cl.: A61K 31/337

(54) **INJECTABLE COMPOSITION FOR THE TREATMENT OF CANCERS**
INJIZIERBARE ZUSAMMENSETZUNG ZUR BEHANDLUNG VON KREBS
COMPOSITION INJECTABLE POUR LE TRAITEMENT DE CANCERS

(30) Priority: 09.04.2004 KR 2004024575
(43) Date of publication of application: 20.12.2006
(73) Proprietor: Jee, Ung-Kil, Seocho-gu, Seoul 137-907 (KR); Park, Jin-Kyu, 726, Seongbok-dong, Yongin-si Gyeonggi-do 449-531 (KR)
(72) Inventor: Jee, Ung-Kil, Seocho-gu, Seoul 137-907 (KR); Park, Jin-Kyu, 726, Seongbok-dong, Yongin-si Gyeonggi-do 449-531 (KR)
(74) Representative: Corizzi, Valérie
(86) International application number: PCT/KR2004/001428
(87) International publication number: WO 2005/097105

(56) References cited:
- EP-A1- 0 705 601
- US-A1- 2002 156 124
- US-A1- 2004 067 919

## Description

### Technical Field

The present invention relates to an injectable composition for the treatment of cancers comprising the poorly water-soluble anticancer drug paclitaxel. More particularly, the present invention relates to an injectable composition for the treatment of cancers comprising the poorly water-soluble anticancer drug paclitaxel which allows the anticancer drug to exhibit improved solubility as well as greatly reduced toxicity, while minimizing the decomposition and the titer loss of active ingredients and preventing the formation of precipitates upon dilution for formulation. The injectable composition is very useful as an injection for intravenous administration.

### Background Art

Paclitaxel, as a representative anticancer drug, is a highly water-insoluble alkaloid extracted from the bark of a western yew tree. Paclitaxel is an antimicrotubule agent which promotes the formation of microtubles from a tubulin dimmer and stabilizes the microtubles against depolymerization. Further, paclitaxel exerts superior anticancer effects against ovarian cancer, breast cancer, head and neck cancer, non-small cell lung cancer and the like.

Paclitaxel has a very low solubility (about 30µg/ml) in water and is physically unstable. Thus, a number of studies regarding these problems of paclitaxel are now being actively undertaken. In particular, paclitaxel has a difficulty in the formulation into an injection due to its poor solubility. In addition, conventional paclitaxel injections have many problems in terms of poor stability of anticancer drugs and high toxicity of solubilizers used. To solve these problems, much research is being actively conducted.

### Disclosure of Invention

### Technical Problem

Publication PCT/AU93/00599 discloses a method for preparing a paclitaxel injection having a pH of 8.1 or lower by adding an organic acid to paclitaxel in order to improve the stability of an anticancer drug. According to this method, the polyoxyethylene castor oil derivative (Cremophor EL TM, hereinafter abbreviated as 'Cremophor EL') used as a solubilizer causes a serious hypersensitivity, and separates a plasticizer from the polyvinyl chloride resin set. Because of these side effects, the injectable composition of paclitaxel is not preferred as an injection.

In order to overcome the above-mentioned problems without the use of Cremophor EL, a liposome formulation is prepared by using phosphatidylcholine as a solubilizer. However, there still remains a problem of low solubility (0.8mg/ml), and the stability of the liposome formulation is not satisfactory. Accordingly, there is a difficulty in mass-production [Pharm. Res., 1994, 11(2), 206∼212; Pharm. Res., 1994, 11(6), 889∼896].

In recent years, a number of studies to improve the solubility of paclitaxel by chemically binding paclitaxel to a biodegradable block copolymer consisting of a hydrophilic segment and a hydrophobic segment have been ongoing. However, this method has disadvantages in terms of complex processes, such as evaporation and lyophilization, during preparation of the copolymer, increased production costs and decreased biocompatibility [JP 116,082; JP 206, 815/94; EP 0 583 955A2].

A preparation commercially available under the brand name of 'Taxol' is known. Taxol is a liquid wherein 30mg of paclitaxel is dissolved in 5ml of a mixed solution of an absolute alcohol and Cremophor EL (1:1). For administration, the liquid preparation is diluted in physiological saline or a 5% glucose solution to a concentration of 0.6∼1.2mg/ml. A dose of 175mg/m³of the dilution is administered intravenously over 6-24 hours. However, since the Cremophor EL as a solubilizer itself is toxic, it causes serious toxicity, including hypersensitivity, dyspnea, flushing, etc., after administration to humans. For this reason, for example, in order to minimize the hypersensitivity caused upon administration of taxol, a drenocortical hormone (Dexamethasone), anti-histimines (Diphenhydramine) and H2-antagonist (Cimetidine) are previously administered. In addition, there is a problem of titer loss caused by the degradation of paclitaxel, resulting from instable preparation. Taxol is required to be stored at low temperature. In addition, various side effects are reported, such as the need of an additional filtering process before administration to humans due to the formation of fine particles with the passage of time.

### Technical Solution

Therefore, the present invention has been made in view of the above problems, and it is one object of the present invention to provide an injectable composition according to claim 1 for the treatment of cancers comprising the poorly water-soluble anticancer drug paclitaxel which allows the anticancer drug to exhibit improved solubility as well as greatly reduced toxicity.

It is another object of the present invention to provide an injectable composition for the treatment of cancers which minimizes the decomposition and the titer loss of active ingredients and prevents the formation of precipitates upon dilution for formulation, thereby being very effectively used as an injection for intravenous administration.

In order to accomplish the above objects of the present invention, there is provided an injectable composition for the treatment of cancers comprising a poorly water-soluble anticancer drug, and glycofurol and Solutol HS15 as solubilizers of the anticancer drug.

According to one preferred embodiment of the present invention, the anticancer drug is paclitaxel.

According to another preferred embodiment of the present invention, the injectable composition for the treatment of cancers has a pH of 4-6.

According to another preferred embodiment of the present invention, the injectable composition for the treatment of cancers further comprises a stabilizer.

According to another preferred embodiment of the present invention, the injectable composition for the treatment of cancers further comprises an acid and/or a buffer for pH adjustment.

According to another preferred embodiment of the present invention, the stabilizer is at least one compound selected from D-α-tocopherol, sodium bisulfite and EDTA (sodium salt).

According to another preferred embodiment of the present invention, the D-α-tocopherol is present in an amount of 1∼10% by weight based on the total weight of the injectable composition.

According to another preferred embodiment of the present invention, the sodium bisulfite or EDTA (sodium salt) is present in an amount of 0.005∼0.05% by weight based on the total weight of the injectable composition. The sodium bisulfite and the EDTA (sodium salt) may be added alone or in combination.

According to another preferred embodiment of the present invention, the buffer is an acetate buffer solution at pH 4-6.

According to another preferred embodiment of the present invention, the buffer is an acetate buffer solution at pH 4-5 containing 5∼20% (w/v) of trehalose.

According to yet another preferred embodiment of the present invention, the glycofurol and the Solutol HS 15 as solubilizers are present in a weight ratio of 20∼40%:10∼40%, based on the total weight of the injectable composition.

### Description of Drawings

The above and other objects, features and other advantages of the present invention will be more clearly understood from the following detailed description taken in conjunction with the accompanying drawings, in which:
Fig. 1 is a histogram showing the particle size distribution of a composition prepared in Example 1 of the present invention;
Fig. 2 is a histogram showing the particle size distribution of a composition prepared in Example 2 of the present invention;
Fig. 3 is a histogram showing the particle size distribution of a composition prepared in Example 3 of the present invention;
Fig. 4 is a histogram showing the particle size distribution of a composition prepared in Example 4 of the present invention;
Fig. 5 is a histogram showing the particle size distribution of a composition prepared in Example 5 of the present invention;
Fig. 6 is a histogram showing the particle size distribution of a composition prepared in Example 6 of the present invention; and
Fig. 7 is a graph showing the pharmacokinetics of compositions prepared in Examples 1 to 3 in rats.

### Best Mode

Hereinafter, the present invention will be explained in more detail.

The term 'poorly water-soluble anticancer drug' as used herein refers collectively to anticancer drugs having a low water solubility so that they are difficult to be used as injections without any particular processing, and is intended to include already known anticancer drugs having a water solubility not higher than 30µg/ml and new anticancer drugs to be developed in the near future. Specific examples of these anticancer drugs include paclitaxel, fluorouracil, docetaxel, etoposide, lomustine, melphalan , mercaptopurine and the like, and paclitaxel is preferred.

The injectable composition of the present invention comprises particular solubilizers for improving the water solubility of the anticancer drug. In the present invention, glycofurol and Solutol HS15 are used as the solubilizers. Specifically, the glycofurol (tetrahydrofurfuryl alcohol polyethyleneglycol ether) is a solution adjuvant, and the Solutol HS15 (polyethylene 660 12-hydroxystearate) is a surfactant.

Glycofurol is a hydrophilic organic solvent, has no toxicity, and is highly suitable for the solubilization of the poorly water-soluble anticancer drug. Glycofurol is comm ercially available, for example, Glycofurol 75 manufactured by Sigma Corporation.

The Solutol HS15 is a surfactant manufactured by BASF Corporation under the brand name of Solutol HS 15, which contains about 30% polyethyleneglycol and about 70% polyethyleneglycol ester. The Solutol HS15 is non-toxic and plays a roll in effectively solubilizing the poorly water-soluble anticancer drug when used in combination with the glycofurol.

An injection is formulated in accordance with the following procedure. First, Solutol HS15 is dissolved in an aqueous medium at elevated temperature with stirring to obtain a first solution. A poorly water-soluble anticancer drug is dissolved in glycofurol to obtain a second solution. At this time, a stabilizer is preferably added to the first solution and/or the second solution. The choice of suitable stabilizers is dependent on the solubilization characteristics. Examples of stabilizers that can be added to the first solution in the aqueous phase include sodium bisulfite, EDTA (sodium salt) and the like. Examples of stabilizers that can be added to the second solution in the oily phase include oleic acid, oily D-α-tocopherol and the like. Sodium bisulfite is a hydrophilic reducing antioxidant, and EDTA (sodium salt) is a stabilizer fixing a slight amount of metal ions, such as iron and copper ions, to form a chelate and to prevent the anticancer drug from being automatically oxidized, thereby stabilizing the anticancer drug. This stabilization of the anticancer drug enables the prevention of titer loss caused by the degradation of the anticancer drug. In addition, the oleic acid and oily D-α-tocopherol stabilize the anticancer drug through the hydrophobic reducing antioxidation mechanism, thereby preventing titer loss caused by the degradation of the anticancer drug.

The amount of the stabilizer added is varied depending on the kind of the stabilizer. If sodium bisulfite and/or EDTA (sodium salt) is used, the amount is preferably between 0.005 and 0.05 % by weight based on the total weight of the injectable composition. On the other hand, if D-α-tocopherol is used, the amount is preferably in the range of 1∼10% by weight based on the total weight of the injectable composition.

The aqueous medium constituting the first solution may further comprise a buffer for the stabilization of the anticancer drug. In particular, paclitaxel is stable at a pH of 4-6. When triple-distilled water is used as the aqueous medium, the final pH of the formulation is commonly in the range of 5∼7. Accordingly, the buffer can be used in order to adjust the pH to 4-6, and preferably 5-5.5. The buffer is, e.g., an acetate buffer solution at pH 4-6, or an acetate buffer solution at pH 4-5 containing 5∼20% (w/v) of trehalose, which makes the ratio of the aqueous medium relatively low and thus improves the physical stability of the formulation. The content of the aqueous medium in the injectable composition is preferably in the range between 10 and 30% by weight, but is not particularly limited to this range.

The amount of the glycofurol and Solutol HS 15 added is dependent on the kind and content of the anticancer drug used. However, t here are no particular limitations on the mixing ratio between the glycofurol and the Solutol HS 15. The glycofurol and the Solutol HS15 are preferably present in a weight ratio of 20∼40%:10∼40%, based on the total weight of the injectable composition. Within this range, most poorly water-soluble anticancer drugs can be efficiently solubilized.

The first solution is slowly added dropwise to the second solution and then the pH of the mixture is adjusted to the weakly acidic range, preferably 4-6, and more preferably 5-5.5. The pH range of the mixture is varied depending on the kind of the anticancer drugs used. The adjustment of pH to these ranges is to overcome the instability of the anticancer drug with the passage of time, that is, to prevent titer loss caused by the degradation of the anticancer drug. Examples of pH-adjusting agents for adjustment of the acidity of the final solution include, but are not particularly limited to, various acids such as acetic acid, citric acid, phosphoric acid, ascorbic acid, gluconic acid, succinic acid, tartaric acid, lactic acid and salts thereof, and acetic acid is preferred.

The injectable composition for the treatment of cancers is diluted about 5∼20-fold with physiological saline or a 5% glucose solution before clinical administration. Anticancer drugs are required to have uniform stability during storage. In addition, when diluted for clinical administration, anticancer drugs must be physically and stably dispersed without a large change in the particle size distribution even after a given period of time.

As a result of an experiment for stability of paclitaxel, paclitaxel had a residual percentage of not lower than 98% at pH 4-6 for 48 hours, and particularly not lower than 99% around pH 5. Accordingly, paclitaxel is confirmed to be very stable (see, Table 1).

As can be seen from Tables 4 and 5, the injectable composition of the present invention is very stable even after long-term storage of 30 days. It can be also confirmed that the anticancer drug has a residual percentage of approximately 97% or higher at room temperature and 40°C. Accordingly, the anticancer drug is confirmed to be stable according to varying time and temperatures.

In the following examples, the injectable composition was diluted 5-fold with a 5% glucose solution and then the particle size distribution was measured 24 hours after the dilution. As a result, the anticancer drug was physically and stably dispersed without a large change in the particle size distribution even after a given period of time. This result suggests that the injectable composition for the treatment of cancers of the present invention will be very effective for clinical administration.

### Mode for Invention

The present invention will now be described in more detail with reference to the following examples. However, these examples are given for the purpose of illustration and are not to be construed as limiting the scope of the invention.

Examples 1 and 2 : Preparation of nano-dispersion emulsion containing paclitaxel, solution adjuvant and surfactant

### <Example 1>

40g of polyethylene glycol 660 12-hydroxystearate (40wt%, hereinafter referred to as 'Solutol HS 15') was added to 18ml of an acetate buffer solution at pH 4.1, and then dissolved at elevated temperature with stirring to obtain a first solution. Separately, 600mg (0.6wt%) of paclitaxel was dissolved in 34g (34wt%) of tetrahydrofurfuryl alcohol polyethyleneglycol ether (hereinafter referred to as 'glycofurol') to obtain a second solution.

The second solution was slowly added dropwise to the first solution, and 2N acetic acid was added until the final volume reached 100ml. The mixture was heated at 60°C for 10 minutes and stirred for 3 hours to obtain a final solution. The final solution had a pH of 5.14.

### <Example 2>

The procedure of Example 1 was repeated, except that an acetate buffer solution (pH 4.1) containing 20% (w/v) of trehalose was used instead of the acetate buffer solution at pH 4.1. The final solution had a pH of 5.28.

Examples 3 and 4 : Preparation of nano-dispersed emulsion containing paclitaxel, oil, solution adjuvant and surfactant

### <Example 3>

40g (40wt%) of Solutol HS 15 was added to 18ml of an acetate buffer solution at pH 4.1, and then dissolved at elevated temperature with stirring to obtain a first solution. Separately, 600mg (0.6wt%) of paclitaxel was dissolved in a mixed solution of 28g (28wt%) of glycofurol and 6g (6wt%) of oleic acid to obtain a second solution.

The second solution was slowly added dropwise to the first solution, and 2N acetic acid was added until the final volume reached 100ml. The mixture was heated at 60°C for 10 minutes and stirred for 3 hours to obtain a final solution. The final solution had a pH of 5.01.

### <Example 4>

The procedure of Example 3 was repeated, except that an acetate buffer solution (pH 4.1) containing 20% (w/v) of trehalose was used instead of the acetate buffer solution at pH 4.1. The final solution had a pH of 5.28.

Examples 5 and 6 : Preparation of paclitaxel injection containing paclitaxel, stabilizer, solution adjuvant and surfactant

### <Example 5>

40g (40wt%) of Solutol HS 15 was added to 18ml of an acetate buffer solution at pH 4.1, and then dissolved at elevated temperature with stirring to obtain a first solution. Separately, 600mg (0.6wt%) of paclitaxel was dissolved in a mixed solution of 28g (28wt%) of glycofurol and 6g (6wt%) of D-α-tocopherol to obtain a second solution.

The second solution was slowly added dropwise to the first solution, and 2N acetic acid was added until the final volume reached 100ml. The mixture was heated at 60°C for 10 minutes and stirred for 3 hours to obtain a final solution. The final solution had a pH of 5.08.

### <Example 6>

0.01g (0.01wt%) of sidium bisulfite and 0.01g (0.01wt%) of EDTA (sodium salt) were added to 18ml of an acetate buffer solution at pH 4.1, and then 40g (40wt%) of Solutol HS 15 was added thereto. The resulting mixture was dissolved at elevated temperature with stirring to obtain a first solution. Separately, 600mg (0.6wt%) of paclitaxel was dissolved in 34g (34wt%) of glycofurol to obtain a second solution.

The second solution was slowly added dropwise to the first solution, and 2N acetic acid was added until the final volume reached 100ml. The mixture was heated at 60°C for 10 minutes and stirred for 3 hours to obtain a final solution. The final solution had a pH of 5.09.

### <Experimental Example 1> Stability test of paclitaxel according to varying pH values

In order to prepare a paclitaxel injection at a stable pH value, stability test of paclitaxel according to varying pH values was performed. Paclitaxel was completely dissolved using glycofurol and Solutol HS 15, and was then diluted in a McIlvaine buffer at pH 3∼8. The dilution was allowed to stand at 40°C. After 24 and 48 hours, the residual percentages of paclitaxel were measured by HPLC. The results are shown in Table 1 below.

**<Table 1> Stability of paclitaxel at various pH values**

| pH | Initial residual percentage (0 hour)(%) | Residual percentage after 24 hours (%) | Residual percentage after 48 hours (%) |
|---|---|---|---|
| 3 | 100 | 99.239 | 97.509 |
| 4 | 100 | 99.025 | 98.221 |
| 5 | 100 | 99.414 | 99.292 |
| 6 | 100 | 98.765 | 98.183 |
| 7 | 100 | 96.109 | 94.344 |
| 8 | 100 | 77.297 | 57.38 |

As can be seen from Table 1, comparing the residual percentage s of paclitaxel with the passage of time at various pH values, paclitaxel was stable at pH 4-6. It is determined from the result that the pH of a paclitaxel injection is preferably adjusted within the range of 5-5.5 by the addition of an appropriate acid.

### <Experimental Example 2> Measurement of particle size distribution

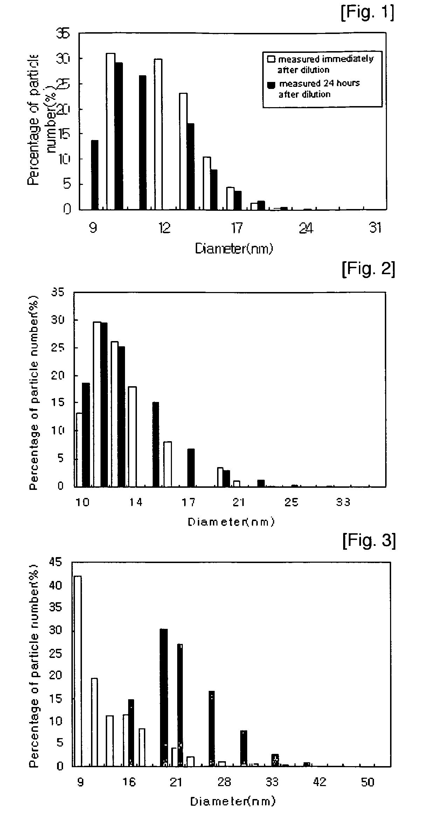

As described above, the injectable composition of the present invention is diluted 5-20-fold with physiological saline or a 5% glucose solution before clinical administration. In this experimental example, the injectable compositions prepared in Examples 1 to 6 were diluted 5-fold with a 5% glucose solution and then the particle size distribution was measured in order to determine the physical stability of paclitaxel in the dilutions, i.e., the formation of precipitates, with the passage of time. After the dilutions were allowed to stand for 24 hours, the particle size distribution was again measured. The results are shown in Table 2 and Figs. 1 to 6.

**<Table 2> Average particle size 0 and 24 hours after dilution**

| Example No. | Average particle size (nm) immediately after dilution | Average particle size (nm) 24 hours after dilution |
|---|---|---|
| 1 | 12.7 | 12.0 |
| 2 | 13.1 | 13.2 |
| 3 | 12.7 | 21.8 |
| 4 | 21.7 | 6880.1 |
| 5 | 15.7 | 16.3 |
| 6 | 12.5 | 13.5 |

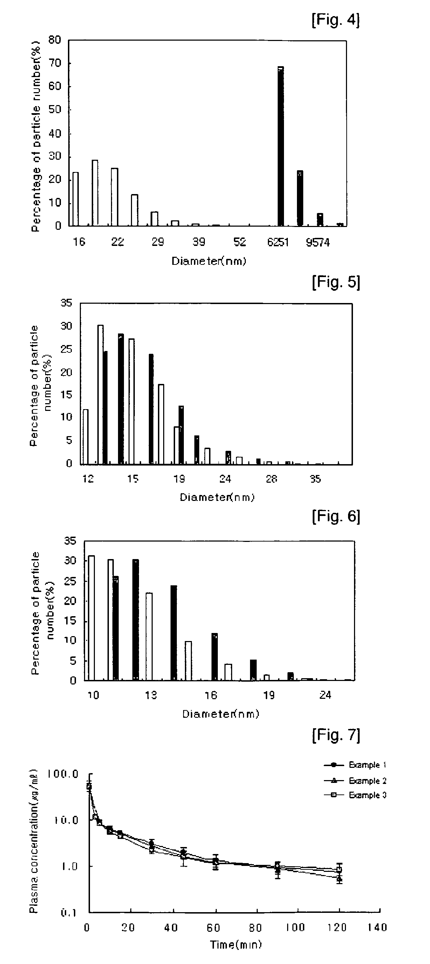

As is evident from Table 2 and Figs. 1 to 6, as the particle size is increased after dilution, the agglomeration and precipitation of particles take place due to decreased stability in the solutions, causing further increase in the particle size. Except in the injectable composition of Example 4 in which oleic acid and trehalose were included, there were no great change in the particle size distribution of the injectable compositions of Examples 1, 2, 3, 5 and 6 for 24 hours after dilution. Accordingly, it can be confirmed that the injectable composition of the present invention is physically and stably dispersed for a given period of time after being diluted for administration. It is assumed that the increase in the particle size of the injectable composition of Example 4 is attributed to the decrease in the solubility of paclitaxel due to the addition of the oleic acid and trehalose in the oily state. <Experimental Example 3> Pharmacokinetics of paclitaxel injectable composition using white rats SD white rats (male, body weight: 230∼260g) were used to test the pharmacokinetics of the paclitaxel injectable compositions. A PE-10 tube was cannulated through the femoral vein of each rat. After 5mg/kg of each of the formulations prepared in Examples 1 to 3 was injected into the rats through the PE-10 tube, blood samples were drawn at various time points. The blood concentration of paclitaxel was analyzed. The experimental results are shown in Table 3 and Fig. 5.

**<Table 3> Pharmacokinetics parameter**

| Example No. | V_{d} (ml) | K₁₀ (l/min) | T_{1/2β} (min) | AUC (µg·min./ml) | Cl (ml/min) |
|---|---|---|---|---|---|
| 1 | 23.343±1.375 | 0.130±0.023 | 36.586±5.664 | 404.727±73.069 | 3.027±0.532 |
| 2 | 22.053±3.709 | 0.145±0.016 | 34.901±3.935 | 381.203±27.705 | 3.159±0.224 |
| 3 | 21.903±1.383 | 0.144±0.031 | 42.795±12.771 | 386.586±59.042 | 3.141±0.480 |

### <Experimental Example 4> Measurement of change in residual percentage of paclitaxel injections with time

The residual percentage of the paclitaxel injectable compositions prepared in Examples 1 to 3 was measured after long-term storage. Specifically, each of the paclitaxel injectable compositions was allowed to stand at room temperature (Table 4) and 40°C (Table 5) for 0, 6, 13, 20 and 30 days of storage . The residual percentage of the compositions was measured by HPLC. The measurement results are shown in Tables 4 and 5 below.

**<Table 4> Residual percentage of paclitaxel at room temperature (%, n=5)**

| Example No. | Initial | After 6 days | After 13 days | After 20 days | After 30 days |
|---|---|---|---|---|---|
| 1 | 100 | 99.947±0.402 | 98.743±0.293 | 97.447±0.434 | 98.891±0.23 |
| 2 | 100 | 100.216±0.320 | 99.110±0.425 | 98.707±0.545 | 99.688±0.435 |
| 3 | 100 | 99.930±0.37 9 | 99.731±0.52 9 | 98.283±0.42 | 100.154±0.340 |

**<Table 5> Residual percentage of paclitaxel at 40°C (%, n=5)**

| Example No. | Initial | After 6 days | After 13 days | After 20 days | After 30 days |
|---|---|---|---|---|---|
| 1 | 100 | 99.101±0.320 | 98.337±0.235 | 97.566±0.159 | 97.696±0.321 |
| 2 | 100 | 98.985±0.534 | 97.845±0.485 | 97.121±0.079 | 98.362±0.279 |
| 3 | 100 | 100.034±0.513 | 98.847±0.138 | 98.475±0.550 | 99.361±0.287 |

As can be seen from Tables 4 and 5, all paclitaxel injectable compositions according to the present invention are very stable both at room temperature and 40°C.

### Industrial Applicability

As apparent from the above description , since the injectable composition according to the present invention comprises a poorly water-soluble anticancer drug and suitable pharmaceutically allowable low-toxicity solubilizers, it exhibits improved solubility as well as greatly reduced toxicity. In addition, since the injectable composition according to the present invention further comprises an acid and a buffer for pH adjustment to the optimum range, and a stabilizer, it can minimize the decomposition and the titer loss of active ingredients and prevent the formation of precipitates upon dilution for formulation. Therefore, the injectable composition of the present invention is very useful as an injection for intravenous administration.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. An injectable composition for the treatment of cancers, comprising:
a poorly water-soluble paclitaxel as an anticancer drug;
glycofurol and a surfactant as solubilizers of said paclitaxel, wherein said surfactant contains 30% polyethyleneglycol and 70% polyethyleneglycol ester by weight based on the total weight of said surfactant;
at least one compound selected from D-α-tocopherol, sodium bisulfite and EDTA (sodium salt) as a stablilizer; and
a buffer for pH adjustment, wherein said buffer is an acetate buffer solution at pH 4∼6 or an acetate buffer solution at pH 4∼5 containing 5∼20% (w/v) of trehalose.

2. The injectable composition according to claim 1, wherein said injectable composition has a pH of 4∼6.

3. The injectable composition according to claim 1 or 2, wherein said D-α-tocopherol is present in an amount of 1∼10% by weight based on the total weight of said injectable composition.

4. The injectable composition according to claim 1, 2 or 3, wherein said sodium bisulfite or said EDTA (sodium salt) is present in an amount of 0.005∼0.05% by weight based on the total weight of the injectable composition, and said sodium bisulfite and said EDTA(sodium salt) is added alone or in combination.

5. The injectable composition according to claim 1, 2, 3 or 4, wherein said glycofurol and said surfactant are present in a weight ratio of 20∼40%:10∼40%, based on the total weight of said injectable composition.

## Patentansprüche

1. Injizierbare Zusammensetzung für die Behandlung von Krebsarten, umfassend:
ein in Wasser schlecht lösliches Paclitaxel als ein Wirkstoff gegen Krebs;
Glycofurol und ein Surfactant als Solubilisatoren des Paclitaxel, wobei das Surfactant 30 Gew.-% Polyethylenglycol und 70 Gew.-% Polyethylenglycolester, bezogen auf das Gesamtgewicht des Surfactants, enthält;
wenigstens eine Verbindung, ausgewählt aus D-α-Tocopherol, Natriumbisulfit und EDTA (Natriumsalz), als Stabilisator und einen Puffer zur pH-Einstellung, wobei der Puffer eine Acetatpufferlösung mit pH 4-6 oder eine Acetatpufferlösung mit pH 4-5 ist, die 5-20% (G/V) Trehalose enthält.

2. Injizierbare Zusammensetzung gemäß Anspruch 1, wobei die injizierbare Zusammensetzung einen pH von 4-6 hat.

3. Injizierbare Zusammensetzung gemäß Anspruch 1 oder 2, wobei das D-α-Tocopherol in einer Menge von 1-10 Gew.-%, bezogen auf das Gesamtgewicht der injizierbaren Zusammensetzung, vorliegt.

4. Injizierbare Zusammensetzung gemäß Anspruch 1, 2 oder 3, wobei das Natriumbisulfit oder das EDTA (Natriumsalz) in einer Menge von 0,005-0,05 Gew.-%, bezogen auf das Gesamtgewicht der injizierbaren Zusammensetzung, vorliegt und das Natriumbisulfit und das EDTA (Natriumsalz) allein oder in Kombination zugesetzt werden.

5. Injizierbare Zusammensetzung gemäß Anspruch 1, 2, 3 oder 4, wobei das Glycofurol und das Surfactant in einem Gewichtsverhältnis von 20-40%:10-40%, bezogen auf das Gesamtgewicht der injizierbaren Zusammensetzung, vorliegen.

## Revendications

1. Composition injectable destinée au traitement de cancers, comprenant :
un paclitaxel médiocrement hydrosoluble en tant que médicament anticancéreux ;
du glycofurol et un surfactant en tant que solubilisants dudit paclitaxel, où ledit surfactant contient 30 % de polyéthylène glycol et 70 % d'ester de polyéthylène glycol en poids en se basant sur le poids total dudit surfactant ;
au moins un composé choisi parmi le D-α-tocophérol, le bisulfite de sodium et l'EDTA (sel de sodium) en tant que stabilisant ; et
un tampon pour l'ajustement du pH, où ledit tampon est une solution de tampon acétate à pH 4∼6 ou une solution de tampon acétate à pH 4∼5 contenant 5∼20 % (p/v) de tréhalose.

2. Composition injectable selon la revendication 1, où ladite composition injectable a un pH de 4∼6.

3. Composition injectable selon la revendication 1 ou 2, dans laquelle le D-α-tocophérol est présent dans une quantité de 1∼10 % en poids en se basant sur le poids total de ladite composition injectable.

4. Composition injectable selon la revendication 1, 2 ou 3, dans laquelle ledit bisulfite de sodium ou ledit EDTA (sel de sodium) est présent dans une quantité de 0,005∼0,05 % en poids en se basant sur le poids total de la composition injectable, et ledit bisulfite de sodium et ledit EDTA (sel de sodium) est ajouté seul ou en combinaison.

5. Composition injectable selon la revendication 1, 2, 3 ou 4, dans laquelle ledit glycofurol et ledit surfactant sont présents dans un rapport pondéral de 20∼40 %/10∼40 %, en se basant sur le poids total de ladite composition injectable.
